# EUROPÄISCHE PATENTSCHRIFT

(11) **EP 3 429 654 B1**
(45) Veröffentlichungstag und Bekanntmachung des Hinweises auf die Patenterteilung: **11.11.2020**
(21) Anmeldenummer: 17710158.1
(22) Anmeldetag: 02.03.2017
(51) Int. Cl.: A61M 1/00, A61M 1/06, F04B 39/00

(54) **MEDIZINISCHE SAUGPUMPE**
MEDICAL SUCTION PUMP
POMPE ASPIRANTE MEDICALE

(30) Priorität: 17.03.2016 EP 16160970
(43) Veröffentlichungstag der Anmeldung: 23.01.2019
(73) Patentinhaber: Medela Holding AG, 6340 Baar (CH)
(72) Erfinder: BÄCHLER, Cornel, 6038 Giskon (CH); FELBER, Armin, 6003 Luzern (CH); MUTHER, Marcel, 6030 Ebikon (CH)
(74) Vertreter: Clerc, Natalia
(86) Internationale Anmeldenummer: PCT/EP2017/054970
(87) Internationale Veröffentlichungsnummer: WO 2017/157691

(56) Entgegenhaltungen:
- WO-A1-2007/122578
- WO-A1-2015/109934
- WO-A1-2016/103031
- DE-A1-102012 101 642
- US-A1- 2008 275 386

## Beschreibung

### TECHNISCHES GEBIET

Die vorliegende Erfindung betrifft eine medizinische Saugpumpe, insbesondere eine Brustpumpe zum Abpumpen von menschlicher Muttermilch oder eine Drainagepumpe zum Absaugen von Körperflüssigkeiten, beispielsweise für die Thoraxdrainage oder für die Wunddrainage.

### STAND DER TECHNIK

Medizinische Saugpumpen, auch Vakuumpumpen genannt, sind für die verschiedensten Anwendungen bekannt. Beispielsweise werden sie als Brustpumpen zum Abpumpen von menschlicher Muttermilch oder Drainagepumpen zum Absaugen von Körperflüssigkeiten verwendet. Derartige Saugpumpen weisen als Pumpaggregat Kolbenpumpen oder Membranpumpen auf. Die Verwendung einer Pumpmembran ermöglicht es, die Saugpumpe relativ klein und leicht und somit im Gebrauch tragbar zu gestalten. Ein sehr kleines und trotzdem die hohen Anforderungen an eine Brustpumpe erfüllendes Pumpaggregat ist beispielsweise in US 2007/0292276 offenbart.

Viele dieser Pumpen haben den Nachteil, dass sie relativ laut sind. Sie lassen sich kaum diskret in der Öffentlichkeit verwenden und/oder stören den Benützer durch die rhythmisch wiederkehrenden Geräusche.

WO 2015/109934 A1 offenbart eine Brustpumpe mit einer Schalldämpfung. Das Pumpaggregat ist in einem geschlossenen Innengehäuse gehalten, wobei der Hohlraum zwischen Gehäuseinnenwand und Pumpaggregat mit Stossdämpfern aus Schaumstoff ausgefüllt ist. Das Innengehäuse weist zwei Hälften auf, welche über einen Silikonring miteinander verbunden sind. Dieses Innengehäuse ist mit Schrauben in einem Brustpumpengehäuse fixiert, wobei drei Stossdämpfer, welche auf der Aussenseite des Innengehäuses angeordnet sind, die Übertragung der Vibrationen des Pumpaggregats auf das äussere Pumpengehäuse verringern. Diese vollständige Umhüllung des Pumpaggregats mit Schaumstoff hat den Nachteil, dass im Aggregat erzeugte Wärme nur sehr schlecht abgeführt werden kann. Zudem benötigt dieses Schaumstoffpolster relativ viel Platz und führt nur zu einer mässigen Körperschalldämpfung.

KR 101462340 B1 beschreibt eine Brustpumpe mit einer Schalldämpfung. Ein metallenes Pumpengehäuse ist mit einer schalldämpfenden Matte ausgelegt und das Pumpaggregat ist passgenau eingelegt. Zwischen Deckelteil und Bodenteil des Gehäuses ist ebenfalls ein schalldämpfender Rand vorgesehen. CN 202342540 U zeigt eine vertikale Anordnung des Pumpaggregats mit einem am Vakuumanschluss des Pumpaggregats angeordneten Dämpfungselement. US 2008/0275386 A1 offenbart eine unter einem Büstenhalter tragbare Brustpumpe mit einem Pumpaggregat. WO 2007/122578 A1 und DE 10 2012 101 642 A1 offenbaren Medizinalpumpen mit einem Pumpengehäuse und einem darin gehaltenen Pumpaggregat.

### DARSTELLUNG DER ERFINDUNG

Es ist deshalb eine Aufgabe der Erfindung, eine medizinische Saugpumpe zu schaffen, welche möglichst geräuscharm betrieben werden kann und welche trotzdem eine möglichst grosse Flexibilität in der Gestaltung der Pumpe ermöglicht.

Diese Aufgabe löst eine medizinische Saugpumpe mit den Merkmalen des Anspruchs 1.

Die erfindungsgemässe medizinische Saugpumpe weist ein Pumpengehäuse, ein im Pumpengehäuse angeordnetes Pumpaggregat zur Erzeugung eines Unterdrucks und eine Einrichtung zur Schalldämpfung auf. Die Einrichtung zur Schalldämpfung weist mindestens zwei elastische Lager zur elastischen Halterung des Pumpaggregats relativ zum Pumpengehäuse auf, welche beabstandet zueinander angeordnet sind.

Die elastische Aufhängung und Lagerung verhindert eine Übertragung von Vibrationen und Körperschall vom Pumpaggregat zum Pumpengehäuse. Das Pumpaggregat ist dank der Erfindung definiert gehalten und trotzdem so flexibel aufgehängt, dass die Geräuschentwicklung reduziert und/oder gedämpft wird.

Die Zwei-, Drei- oder Mehrpunktlagerung an voneinander beabstandeten und somit diskreten Stellen in Bezug auf das Pumpaggregat ohne weitere dazwischen liegende Schalldämpfungselemente hat den Vorteil, dass die Halterung des Pumpaggregats innerhalb des Pumpengehäuses relativ frei gewählt werden kann. Die Verwendung von genau drei Lagern bildet ein statisch bestimmtes System.

Die Schalldämpfung ist verbessert, wenn das Pumpaggregat zusätzlich in einem Schalldämpfungsgehäuse angeordnet ist und relativ zu diesem elastisch gelagert ist. Das Schalldämpfungsgehäuse bildet eine abgeschlossene Hülle und reduziert so die Ausbreitung von Luftschall. Vorzugsweise weist es ein Puffervolumen für aus dem Pumpaggregat austretende Abluft auf. Ein derartiges Puffervolumen reduziert den Strömungsdruck und Druckstösse und vermindert somit die Geräuschentwicklung.

Damit Pumpaggregate bekannter Art unverändert verwendet werden können, ist vorzugsweise ein Pumpaggregat-Träger vorhanden, welcher die elastischen Lager trägt und welcher das Pumpaggregat lagefixiert hält. Je nach Ausführungsform bildet der Pumpaggregat-Träger eine Einheit oder er ist aus zwei oder mehr voneinander beabstandeten Untereinheiten oder Trägerteilen gebildet.

Vorzugsweise ist das Pumpaggregat mit Vorspannung in diesem Träger gehalten, damit die Verbindung zwischen den zwei Hauptteilen des Pumpaggregats, nämlich Motor und Vakuumaggregat, möglichst fest ist und nicht zu Vibrationen führt. In bevorzugten Ausführungsbeispielen ist der Pumpaggregat-Träger hierfür als eine zusammenhängende Einheit ausgebildet. Diese Vorspannung reduziert die Geräuschentwicklung des Aggregats merklich.

Vorzugsweise sind genau zwei elastische Lager vorhanden und das Pumpaggregat ist ausschliesslich über diese zwei Lager mit dem

Schalldämpfungsgehäuse verbunden. Vorzugsweise befinden sich diese genau zwei Lager an einander entgegengesetzten Enden des Pumpaggregats. Insbesondere befinden sie sich entlang einer Längsachse des Pumpaggregats oder gleichabständig versetzt auf gegenüberliegenden Seiten zu dieser. Vorzugsweise befinden sie sich auf der Schwerpunktsachse des Pumpaggregats. Vorzugsweise sind genau drei elastische Lager vorhanden, wobei die oben genannte Verbindung ausschliesslich über diese drei Lager erfolgt. Dreipunktlagerungen sind definierte Anordnungen und verunmöglichen Spiel der einzelnen Teile.

Vorzugsweise sind die mindestens zwei oder mindestens drei Lager aus einem Elastomer gebildet. Diese Materialien sind kostengünstig, in beliebigen Formen herstellbar und vor allem widerstandsfähig im Gebrauch. Es ist jedoch kaum eine Materialermüdung vorhanden.

Vorzugsweise sind ein erstes und ein zweites dieser Lager in voneinander entgegengesetzte Richtungen ausgerichtet.

Ist ein drittes Lager vorhanden, so ist es vorzugsweise in eine weitere, von den genannten Richtungen senkrecht verlaufende Richtung ausgerichtet.

In Ausführungsformen mit mindestens oder genau drei Lagern sind das erste und das zweite Lager vorzugsweise im Bereich eines Vakuumaggregats des Pumpaggregats angeordnet und das dritte Lager ist vorzugsweise im Bereich eines Motors des Pumpaggregats angeordnet. Eine ausgewogene Lagerung mit Berücksichtigung des Schwerpunkts des Aggregats ist von Vorteil.

In einer bevorzugten Ausführungsform sind die mindestens zwei Lager in Lageraufnahmen einschiebbar gehalten. Dies erleichtert die Herstellung und den Zusammenbau der Pumpe.

Vorzugsweise sind die mindestens zwei Lager aufsteckbare elastomere Körper. Auch dies erleichtert die Herstellung und den Zusammenbau.

Das Schalldämpfungsgehäuse ist vorzugsweise zweiteilig ausgebildet. Vorzugsweise ist es bis auf eine erste und eine zweite Luftdurchlassöffnung sowie bis auf einen Vakuumanschluss luftdicht geschlossen ausgebildet.

Es ist eine Luftaustauscheinheit vorhanden, welche Luft durch die erste Luftdurchlassöffnung des Schalldämpfungsgehäuses dem Pumpaggregat zuführt und Luft durch die zweite Luftdurchlassöffnung des Schalldämpfungsgehäuses aus dem Pumpaggregat abführt. Die Luftaustauscheinheit kann einstückig am Schalldämpfungsgehäuse oder einstückig am Pumpengehäuse ausgebildet sein. Vorzugsweise ist es jedoch ein separates Bauteil, welches zwischen Pumpengehäuse und Schalldämpfungsgehäuse angeordnet ist.

Das Schalldämpfungsgehäuse ist lagefixiert im Pumpgehäuse angeordnet, wobei die Luftaustauscheinheit zwischen Pumpgehäuse und Schalldämpfungsgehäuse angeordnet ist.

In einer bevorzugten Ausführungsform ist die Luftaustauscheinheit plattenförmig ausgebildet.

Vorzugsweise weist die Luftaustauscheinheit Kanäle mit Kanalwänden auf, welche schalldämpfend, vorzugsweise weich, ausgebildet sind. Auch dies erhöht die Schalldämpfung.

Vorzugsweise ist vom Pumpaggregat weggeführte Abluft mindestens über einen Teil eines Strömungsweges durch einen rohrförmigen Luftstrom-Schalldämpfer geleitet. Dies vermindert den Luftschall durch Randreibung der durchströmenden Luft.

Weitere Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Figur 1: eine schematische Darstellung einer erfindungsgemässen Saugpumpe in einer ersten Ausführungsform;
- Figur 2: eine schematische Darstellung eines Pumpaggregats und eines Pumpaggregat-Trägers nach Figur 1;
- Figur 3: eine perspektivische Darstellung des Pumpaggregats gemäss Figur 2;
- Figur 4: eine perspektivische Darstellung des Pumpaggregat-Trägers gemäss Figur 2;
- Figur 5: einen Längsschnitt durch den Pumpaggregat-Träger mit darin gehaltenem Pumpaggregat gemäss Figur 2;
- Figur 6: eine perspektivische Ansicht eines ersten Schalldämpfungs-Gehäuseteils gemäss Figur 1 von unten;
- Figur 7: eine perspektivische Ansicht einer Luftaustauscheinheit gemäss Figur 1 von oben;
- Figur 8: eine schematische Darstellung eines Pumpaggregats und eines Pumpaggregat-Trägers gemäss einer zweiten Ausführungsform der Erfindung;
- Figur 9: eine schematische Darstellung eines Pumpaggregats und eines Pumpaggregat-Trägers gemäss einer dritten Ausführungsform der Erfindung;
- Figur 10: eine perspektivische Ansicht eines ersten Pumpaggregat-Trägerteils gemäss Figur 9 und
- Figur 11: eine perspektivische Ansicht eines zweiten Pumpaggregat-Trägerteils gemäss Figur 9.

### BESCHREIBUNG BEVORZUGTER AUSFÜHRUNGSFORMEN

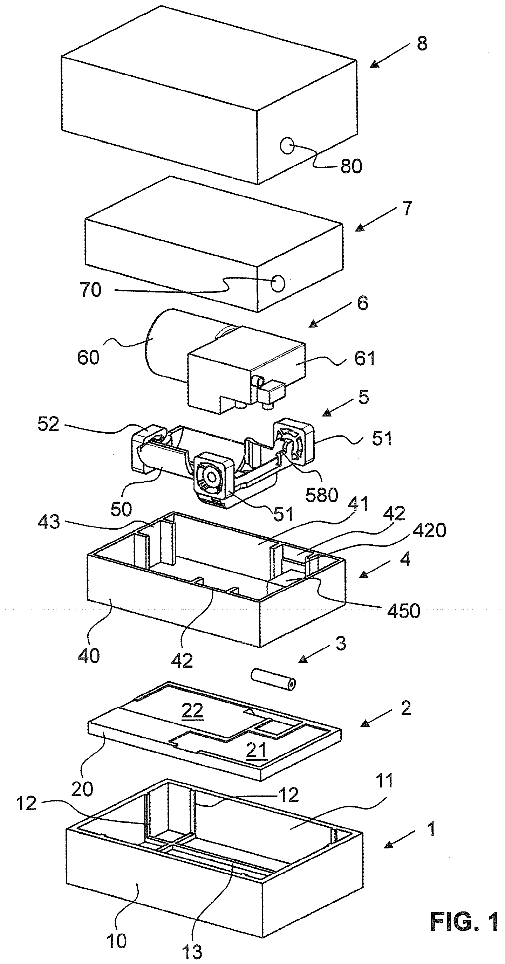

In Figur 1 ist schematisch eine erfindungsgemässe medizinische Saugpumpe in einer Explosionsdarstellung gezeigt.

Die Saugpumpe ist beispielsweise eine Brustpumpe zum Abpumpen von menschlicher Muttermilch. Die Brustpumpe wird direkt oder über einen Vakuumschlauch mit einer oder zwei Brusthauben zur Auflage auf die Mutterbrust verbunden.

Die Saugpumpe ist in einer anderen Ausführungsform eine Drainagepumpe, beispielsweise für die Thoraxdrainage oder Wunddrainage.

Die Saugpumpe ist vorzugsweise relativ klein gestaltet, so dass sie portabel ist und bei Gebrauch am Körper getragen oder in der Hand gehalten werden kann.

Die Saugpumpe umfasst ein Pumpengehäuse mit einem ersten Pumpen-Gehäuseteil 1 und einem zweiten Pumpen-Gehäuseteil 8. In diesem Pumpengehäuse 1, 8 ist ein Schalldämpfungsgehäuse fixiert gehalten, welches vorzugsweise ebenfalls im Wesentlichen aus einem ersten Schalldämpfungs-Gehäuseteil 4 und einem zweiten Schalldämpfungs-Gehäuseteil 7 besteht.

Im Schalldämpfungsgehäuse 4, 7 ist ein Pumpaggregat 6 gelagert, wobei es direkt am Schalldämpfungsgehäuse 4, 7 befestigt sein kann. In diesem Beispiel ist das Pumpaggregat 6 in einem Pumpaggregat-Träger 5 angeordnet, welcher im Schalldämpfungsgehäuse 4, 7 gelagert ist. Die Lagerung des Pumpaggregats 6 bzw. des Pumpaggregat-Trägers 5 erfolgt erfindungsgemäss über mindestens zwei, vorzugsweise genau drei elastische Lager 51, 52. Es sind keine weiteren Befestigungen oder Verbindungen zum Schalldämpfungsgehäuse 4, 7 vorhanden. Diese Aufhängung des Pumpaggregats 6 alleine oder, wie hier dargestellt, gemeinsam mit dem Pumpaggregat-Träger 5 verhindert die Übertragung von Vibrationen auf das Schalldämpfungsgehäuse 4, 7 und somit auf das Pumpengehäuse 1, 8. Die Aufhängung wird weiter unten im Text noch genauer beschrieben.

Das Pumpaggregat 6 ist eine bekannte Einheit zur Erzeugung von Unterdruck und bildet den Kern der Vakuumpumpe. Üblicherweise umfasst das Pumpaggregat 6 einen Motor 60, üblicherweise einen Elektromotor, sowie ein Vakuumaggregat 61. Das Vakuumaggregat 61 weist üblicherweise eine Pumpkammer mit einer Pumpmembran auf, welche über eine Antriebsstange mit dem Motor verbunden ist und mittels des Motors bewegbar ist. Ein derartiges Pumpaggregat ist beispielsweise in US 2007/0292276 beschrieben.

Damit das Pumpaggregat 6 mit der für den Betrieb notwendigen Luftzufuhr und -abfuhr versehen ist, weist die erfindungsgemässe Saugpumpe ein Spaltsystem, hier Luftaustauscheinheit 2 genannt, auf. Die Luftaustauscheinheit 2 ist in diesem Beispiel zwischen dem ersten Pumpen-Gehäuseteil 1 und dem ersten Schalldämpfungs-Gehäuseteil 4 angeordnet und als separates Bauteil ausgebildet. Sie kann jedoch auch integraler Bestandteil eines Pumpen-Gehäuseteils 1, 8 oder eines Schalldämpfungs-Gehäuseteils 4, 7 sein.

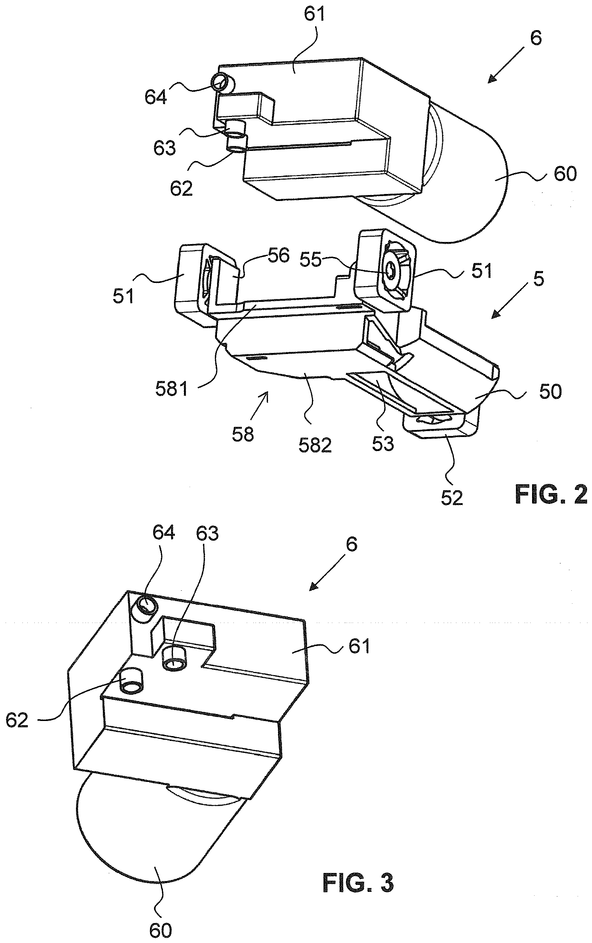

Im Folgenden werden die einzelnen, oben genannten Teile detaillierter beschrieben: Wie in den Figuren 2 und 3 gut erkennbar ist, ist der Motor 60 in diesem Beispiel zylinderförmig liegend ausgebildet. Dies entspricht der üblichen Form. Das damit fest verbundene Vakuumaggregat 61 kann unterschiedliche Formen aufweisen. In diesem Beispiel setzt es sich im Wesentlichen aus zwei Quadern zusammen. Das Vakuumaggregat 61 weist eine Belüftungsöffnung 63, eine Abluftöffnung 62 sowie einen Vakuumanschluss 64 auf. Die Belüftungsöffnung 63, die Abluftöffnung 62 und der Vakuumanschluss 64 sind in diesem Beispiel als Anschlussstutzen ausgebildet. Andere Ausbildungen sind möglich.

Der Pumpaggregat-Träger 5 ist vorzugsweise aus einem steifen Material gefertigt. Vorzugsweise besteht er aus Kunststoff.

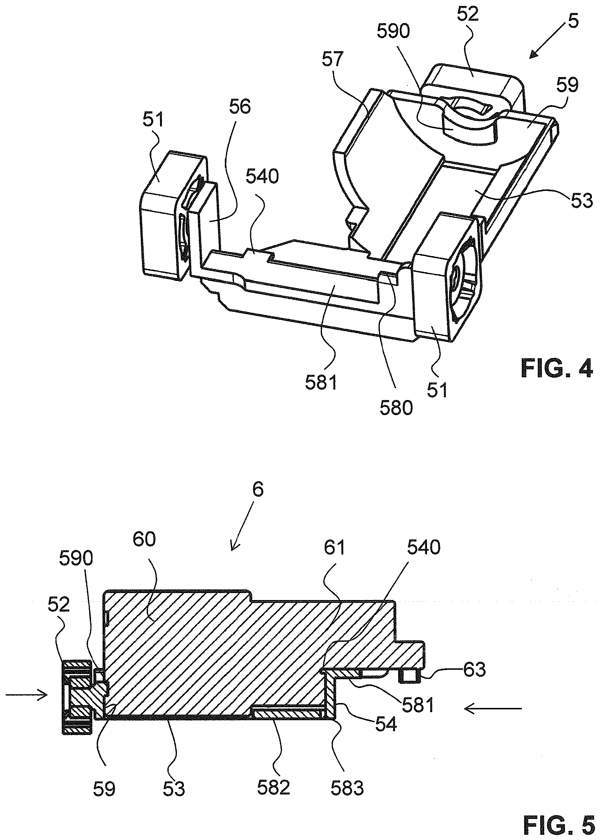

Der Pumpaggregat-Träger 5 ist, wie in den Figuren 2 und 4 gut erkennbar, entsprechend der Form des Pumpaggregats 6 geformt. Er weist eine Motoraufnahme 50 in Gestalt einer halbzylinderförmigen Schale mit einem relativ breiten Spalt 53 auf. Die Motoraufnahme 50 geht auf einer Stirnseite in eine Aggregatauflage 58 über. Diese bildet mit einer der Motoraufnahme 50 zugewandten Fläche einen ersten Anschlag 54, wie dies in Figur 5 gut erkennbar ist. Das dieser Fläche gegenüberliegende Ende der Motoraufnahme 50 bildet einen zweiten Anschlag 59. Der erste Anschlag 54 ist mit mindestens einer ersten Rastnase 540 versehen, der zweite Anschlag 59 mit mindestens einer zweiten Rastnase 590.

Der zur Motoraufnahme 50 hin gerichtete Boden 582 der Aggregatauflage 58 ist in einem Eckbereich mit einem Spalt oder einer Öffnung 583 versehen. Die Öffnung 583 dient der Entformung aus dem Spritzgusswerkzeug, respektive zur Formung der ersten Rastnase 540.

Die Aggregatauflage 58 weist einen von der Motoraufnahme 50 weg gerichteten Balkon 581 zur Auflage des Vakuumaggregats 61 auf. Der Balkon 581 ist an einem nach oben gerichteten, hinteren Anschlag vorzugsweise mit einer dritten Rastnase 580 versehen.

Das Pumpaggregat 6 ist dank der ersten und zweiten Rastnasen 540, 590 und, falls vorhanden, auch dank der dritten Rastnase 580 unter Vorspannung im Pumpaggregat-Träger 5 gehalten. Dadurch werden im Betrieb der Pumpe allfällige Vibrationen zwischen Motor 60 und Vakuumaggregat 61 vermieden und die Geräuschentwicklung minimiert.

Die Aggregatauflage 58 weist auf beiden Seiten je einen nach oben gerichteten seitlichen Anschlag 56 zur Aufnahme des Vakuumaggregats 61 auf, wie dies in Figur 2 erkennbar ist. Auf der Aussenseite dieser Anschläge 56 ist je ein nach aussen gerichteter Befestigungsstift 55 zur Aufnahme je eines ersten Lagers 51 vorhanden. Das Lager 51 ist elastisch ausgebildet. Vorzugsweise ist es ein Elastomerlager. In diesem Beispiel weist jedes Lager 51 einen abgerundeten quaderförmigen Rahmen mit einem mit Stegen versehenen Hohlraum auf. Das Lager 51 lässt sich durch einfaches Aufstecken auf den Befestigungsstift 55 montieren und fixieren.

Am freien Ende der Motoraufnahme 50 ist ein weiterer Befestigungsstift 55 angeformt, welcher ein zweites elastisches Lager 52, vorzugsweise ebenfalls ein Elastomerlager, trägt.

Die drei Lager 51, 52 sind vorzugsweise identisch in Grösse, Form und Elastizität ausgebildet. Sie sind vorzugsweise so angeordnet, dass die bei Bewegung des Pumpaggregat-Trägers 5 gemeinsam mit dem Pumpaggregat 6 wirkenden Kräfte gleichmässig verteilt sind, d.h. dass die Anordnung gleichmässig bezüglich ihres Schwerpunkts gehalten ist.

Wie in Figur 1 dargestellt, ist der Pumpaggregat-Träger 5 gemeinsam mit dem darin unter Vorspannung gehaltenen Pumpaggregat 6 in einem Innenraum 41 des ersten Schalldämpfungs-Gehäuseteils 4 mittels der elastischen Lagerung aufgehängt.

In einer ersten Halbschale 40, welche im Wesentlichen das erste Schalldämpfungs-Gehäuseteil 4 bildet, sind erste Lageraufnahmen 42 für das erste und zweite Lager 51 und eine zweite Lageraufnahme 43 für das dritte Lager 52 vorhanden. Die Lageraufnahmen 42, 43 sind je durch zwei parallele und nach innen ragende Seitenwände gebildet, in welche die Lager 51, 52 von oben her eingeschoben werden können. Die Lager 51, 52 werden dadurch zusammen gepresst und in ihrer Position gehalten. Die ersten Lageraufnahmen 42 weisen zwischen den zwei Seitenwänden je einen unteren Anschlag 420 auf, damit der Pumpaggregat-Träger 5 horizontal in der ersten Halbschale 40 und parallel zu deren Boden angeordnet werden kann. Andere Arten der elastischen Lagerung, der Aufhängung und der Ausgestaltung der Lager in Form und Anordnung sind im Sinne dieser Erfindung möglich.

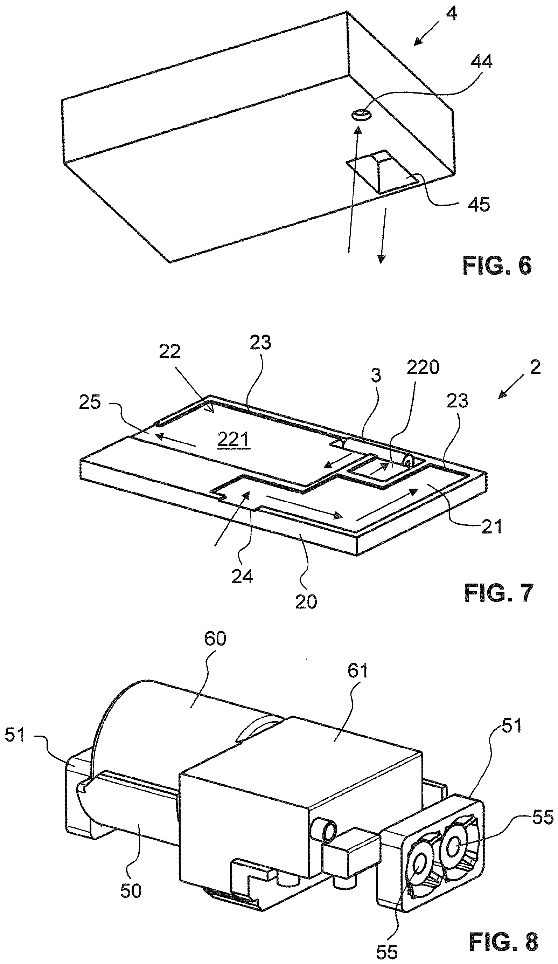

In Figur 8 ist eine alternative Ausführungsform dargestellt. Hier sind genau zwei elastische Lager 51 vorhanden, welche in Längsrichtung des Pumpaggregats 6 an zwei gegenüberliegenden Seiten angeordnet sind. Sie können identisch wie die oben beschriebenen Lager ausgebildet und gehalten sein. Sie können jedoch auch anders ausgebildet oder zumindest grösser sein als die oben erwähnten Lager. Im hier dargestellten Beispiel ist mindestens eines der zwei Lager 51, hier das dem Vakuumaggregat 61 zugewandte Lager 51, breiter ausgebildet. Es weist vorzugsweise zwei Stegstrukturen innerhalb eines geschlossenen Rahmens auf und/oder es ist vorzugsweise an zwei Befestigungsstiften 55 gehalten. Die übrigen Teile entsprechen der Ausführungsform gemäss den Figuren 1 bis 7 und sind hier nicht mehr dargestellt.

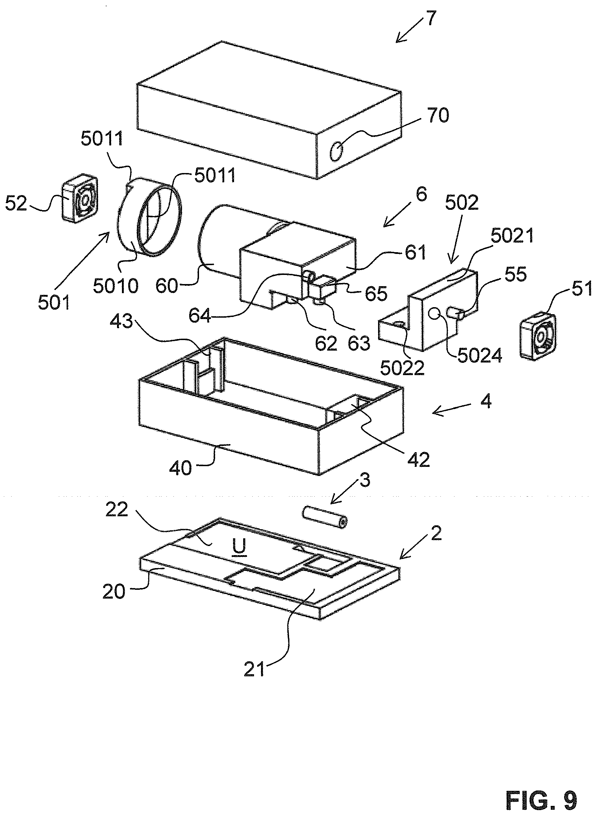

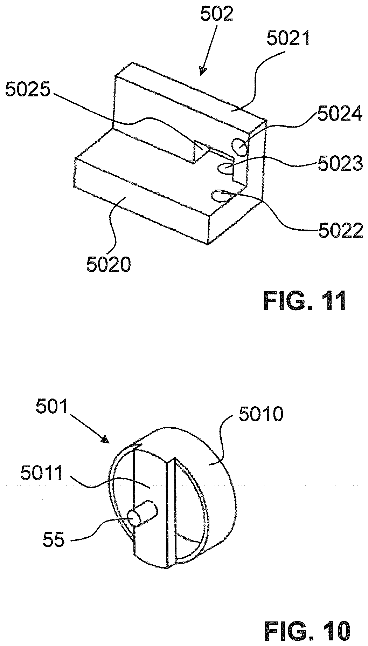

In den Figuren 9 bis 11 ist ein drittes Ausführungsbeispiel der erfindungsgemässen Vorrichtung dargestellt. Gleiche Teile sind mit gleichen Bezugszeichen versehen wie in den oberen Beispielen und werden nicht mehr im Detail erläutert. Das Pumpengehäuse mit dem ersten Pumpen-Gehäuseteil 1 und dem zweiten Pumpen-Gehäuseteil 8 ist hier nicht dargestellt, aber nach wie vor vorhanden.

In dieser dritten Ausführungsform ist der Pumpaggregat-Träger zweiteilig ausgebildet. Er weist ein erstes Pumpaggregat-Trägerteil 501 und ein zweites Pumpaggregat-Trägerteil 502 auf. Sie sind an zwei einander gegenüberliegenden Enden des Pumpaggregats 6 angeordnet.

Das erste Pumpaggregat-Trägerteil 501 weist einen Grundkörper 5010 auf, welcher zur Aufnahme des Motors 60 geeignet ist. Er ist in diesem Beispiel deshalb kreiszylinderförmig ausgebildet. Eine Seite ist offen zur haltenden Aufnahme des Motors 60. Die gegenüberliegende Seite kann geschlossen ausgebildet sein. In diesem Beispiel ist sie teilweise geschlossen ausgebildet, indem ein Steg 5011 sich über die Diagonale erstreckt. Am Steg 5011 ist der vorstehende Befestigungsstift 55, vorzugsweise mittig, angeordnet. Der Befestigungsstift 55 dient zur Halterung des zweiten Lagers 52.

Das zweite Pumpaggregat-Trägerteil 502 ist als rechtwinkliges Winkelelement ausgebildet. Es weist als ersten Schenkel eine Basis 5020 und als zweiten Schenkel einen Anschlag 5021 auf. Das Vakuumaggregat 61 liegt auf der Basis 5020 auf. Die Basis 5020 ist mit einer ersten Durchgangsöffnung 5022 zur Aufnahme des Stutzens der Abluftöffnung 62 sowie mit einer zweiten Durchgangsöffnung 5023 zur Aufnahme des Stutzens der Belüftungsöffnung 63 versehen. Der Anschlag 5021 weist eine dritte Durchgangsöffnung 5024 zur Aufnahme des Stutzens des Vakuumanschlusses 64 sowie eine Ausnehmung 5025 zur Aufnahme einer vorstehenden Nase 65 des Vakuumaggregats 61 im Bereich der Belüftungsöffnung 63 auf.

Die dem Vakuumaggregat 61 abgewandte Seite des Anschlags 5021 ist ebenfalls mit einem Befestigungsstift 55 versehen, welcher der Halterung des ersten Lagers 51 dient.

In diesem Beispiel sind genau zwei Lager 51, 52 vorhanden. Die erste Lageraufnahme 42 und die zweite Lageraufnahme 43 befinden sich entsprechend angeordnet im ersten Schalldämpfungs-Gehäuseteil 4. Dabei liegen sie in diesem Beispiel nicht auf einer Parallelen zur Längsachse des ersten Schalldämpfungs-Gehäuseteils 4, sondern sind so zueinander versetzt angeordnet, dass sie eine möglichst gleichmässige Gewichtsverteilung des Pumpaggregats 6 auf die zwei Lager 51, 52 ermöglichen.

Im Folgenden wird die Erfindung wieder anhand des ersten Ausführungsbeispiels gemäss den Figuren 1 bis 7 erläutert, wobei die Beschreibung auch auf das zweite und dritte Beispiel sowie auf weitere Ausführungsbeispiele im Sinne der Erfindung zutrifft.

Das erste Schalldämpfungs-Gehäuseteil 4 wird mit dem zweiten Schalldämpfungs-Gehäuseteil 7 verschlossen. Der Verschluss erfolgt vorzugsweise derart, dass keinerlei Vibrationen zwischen den Gehäuseteilen 4, 7 erfolgen können oder dass diese zumindest keinen Schall erzeugen können. Ersteres lässt sich beispielsweise durch Verschweissen erzielen, letzteres durch entsprechend weiche und schalldämpfende Verbindung.

Das Schalldämpfungsgehäuse 4, 7 ist vorzugsweise luftdicht verschlossen, wobei eine Durchführungsöffnung für den Saug- oder Vakuumanschluss vorhanden sein muss. Dieser ist in Figur 1 mit dem Bezugszeichen 70 versehen. Er verbindet den Vakuumanschluss 64 des Pumpaggregats 6 über den ebenfalls in Figur 1 sichtbaren Vakuumanschluss 80 des Pumpengehäuses 8 mit einem Saug- oder Vakuumschlauch oder direkt mit einer Brusthaube bzw. einem Fluidsammelbehälter.

Bei den meisten Vakuum-Pumpaggregaten ist zudem eine Luftzufuhr, d.h. eine Belüftung, und eine Luftabfuhr, d.h. ein Auspuff, notwendig. Diese Luft muss somit in das geschlossene Schalldämpfungsgehäuse 4, 7 eingebracht werden bzw. muss aus diesem herausströmen können.

Wie in Figur 6 dargestellt, weist das erste Schalldämpfungs-Gehäuseteil 4 deshalb einen ersten Durchlass 44 für die Zuluft für die Belüftung und eine zweite Durchlassöffnung 45 für die ausströmende Abluft auf. Die zweite Durchlassöffnung 45 ist als erweiterter Pufferraum 450 ausgebildet, wie in Figur 1 erkennbar ist.

Da das Pumpengehäuse 1, 8 üblicherweise nicht luftdicht ausgebildet ist, erübrigen sich spezielle Luftein- und Auslassöffnungen im Pumpengehäuse 1, 8. Sie können jedoch trotzdem vorgesehen sein, falls notwendig oder gewünscht.

Die einströmende und ausströmende Luft kann sich in einer einfachen Ausführungsform ohne weitere Führung zwischen Innenraum 11 des Pumpengehäuses 1, 8 und Schalldämpfungsgehäuse 4, 7 bewegen.

In der hier vorliegenden Ausführungsform ist jedoch die Luftaustauscheinheit 2, auch Spaltsystem genannt, vorhanden. Die Luftaustauscheinheit 2 ist vorzugsweise plattenförmig ausgebildet. Sie ist vorzugsweise steif, insbesondere aus Kunststoff gefertigt. Sie befindet sich auf der Unterseite des ersten Schalldämpfungs-Gehäuseteils 4 und ist luftdicht mit diesem verbunden. Insbesondere ist sie mit diesem verklebt oder verschweisst.

Wie in Figur 7 dargestellt, weist die Luftaustauscheinheit 2 eine schmale, vorzugsweise steife Grundplatte 20 auf, welche mit einem nach oben offenen Belüftungskanal 21 und einem ebenfalls nach oben offenen Abluftkanal 22 versehen ist. Die Kanäle 21, 22 sind durch die Verbindung mit dem in Figur 6 dargestellten ersten Luftdämpfungs-Gehäuseteils 4 verschlossen, wobei der Belüftungskanal 21 in die erste Durchlassöffnung 44 mündet und die zweite Durchlassöffnung 45 in den Abluftkanal 22 führt.

Der Belüftungskanal 21 beginnt an einer Eintrittsöffnung 24, welche durch eine sich bis zum Rand der Grundplatte 20 erstreckende Vertiefung geschaffen ist. Die Austrittsöffnung 25 des Abluftkanals 24 wird gleichermassen, an einer anderen Stelle geschaffen. Die Kanäle 21, 22 sind flächig und weisen somit ein relativ grosses Volumen auf. Zudem ist der Strömungsdruck der Abluft durch den Pufferraum 450 reduziert. Dieser mündet vorzugsweise in den Pufferboden 220.

Vom Pufferboden 220 gelangt die Abluft in einen vertieften Kanal, in welchem ein Luftstrom-Schalldämpfer 3, auch Absorptionsdämpfer genannt, angeordnet ist. Dies ist vorzugsweise ein Silikonschlauch oder -röhrchen, welches von der Abluft durchströmt wird. Der Luftstrom-Schalldämpfer 3 kann in der Luftaustauscheinheit 2 einfach eingelegt sein oder mit dieser fest verbunden, beispielsweise verklebt oder verschweisst sein.

Anschliessend gelangt die Abluft in den Ausströmraum 221, welcher eine relativ grosse Grundfläche aufweist, und von dort durch die Austrittsöffnung nach aussen.

Damit die Geräuschentwicklung minimiert ist, sind mindestens die Seitenwände 23 der zwei Kanäle 21, 22 mit einem weichen Material versehen. Beispielsweise ist eine Silikonschicht auf diese Seitenwände 23 aufgespritzt.

Dieser Luftstrom ist üblicherweise nicht nur für den Betrieb der Pumpe notwendig, sondern trägt auch zur Kühlung des Pumpaggregats bei bzw. er dient als einziges Element der Kühlung.

Das nun mit der Luftaustauscheinheit 2 komplettierte Schalldämpfungsgehäuse 4, 7 wird im Pumpengehäuse 1, 8 angeordnet. Es lässt sich zum Beispiel in ein erstes Pumpen-Gehäuseteil 10 einlegen und auf die übliche Weise fixieren, z.B. indem es verschraubt und/oder eingeklemmt wird. Zusätzlich zum Schalldämpfungsgehäuse 4, 7 sind üblicherweise noch weitere Elemente im Pumpengehäuse 1, 8 angeordnet, welche hier jedoch nicht dargestellt sind. Diese Elemente sind beispielsweise Akkus und/oder Stromwandler und Elektronikbauteile.

Vorzugsweise ist das Pumpengehäuse 1, 8 mit seitlichen und unteren Auflagerippen 12, 13 und gegebenenfalls auch mit oberen Abstandrippen versehen, damit das Schalldämpfungsgehäuse 4, 7 distanziert von den Seitenwänden des Pumpengehäuses gehalten ist.

Das Pumpengehäuse 1, 8 kann jede beliebige Form aufweisen. Auch das Schalldämpfungsgehäuse 4, 7 kann beliebig geformt sein. Pumpengehäuse 1, 8 und Schalldämpfungsgehäuse 4, 7 sind beide vorzugsweise aus einem steifen Material, insbesondere aus Kunststoff, gefertigt.

Die erfindungsgemässe Saugpumpe ermöglicht dank der flexiblen Lagerung eine optimale Schalldämpfung.

**BEZUGSZEICHENLISTE**

| | | | |
|---|---|---|---|
| 1 | erstes Pumpen-Gehäuseteil | 50 | Motoraufnahme |
| 10 | erste Gehäuse-Halbschale | 501 | erstes Pumpaggregat-Trägerteil |
| 11 | Innenraum | | |
| 12 | seitliche Auflagerippe | 5010 | Grundkörper |
| 13 | untere Auflagerippe | 5011 | Steg |
| | | 502 | zweites Pumpaggregat-Trägerteil |
| 2 | Luftaustauscheinheit | | |
| 20 | Grundplatte | 5020 | Basis |
| 21 | Belüftungskanal | 5021 | Anschlag |
| 22 | Abluftkanal | 5022 | erste Durchgangsöffnung |
| 220 | Pufferboden | 5023 | zweite Durchgangsöffnung |
| 221 | Ausströmraum | 5024 | dritte Durchgangsöffnung |
| 23 | Kanalwand | 5025 | Ausnehmung |
| 24 | Eintrittsöffnung | 51 | erstes Lager |
| 25 | Austrittsöffnung | 52 | zweites Lager |
| | | 53 | Spalt |
| 3 | Luftstrom-Schalldämpfer | 54 | erster Anschlag |
| | | 540 | erste Rastnase |
| 4 | erstes Schalldämpfungs-Gehäuseteil | 55 | Befestigungsstift |
| | | 56 | seitlicher Anschlag |
| 40 | erste Schalldämpfer-Halbschale | 58 | Aggregatauflage |
| | | 580 | Rastnase |
| 41 | Innenraum | 581 | Balkon |
| 42 | erste Lageraufnahme | 582 | Boden |
| 420 | Anschlag | 583 | Öffnung |
| 43 | zweite Lageraufnahme | 59 | zweiter Anschlag |
| 44 | erste Luft-Durchlassöffnung | 590 | zweite Rastnase |
| 45 | zweite Luft-Durchlassöffnung | | |
| 450 | Pufferraum | 6 | Pumpaggregat |
| | | 60 | Motor |
| 5 | Pumpaggregat-Träger | 61 | Vakuumaggregat |
| 62 | Abluftöffnung | | Gehäuseteil |
| 63 | Belüftungsöffnung | 70 | Vakuumanschluss |
| 64 | Vakuumanschluss | | |
| 65 | Nase | 8 | zweites Pumpen-Gehäuseteil |
| | | 80 | Vakuumanschluss |
| 7 | zweites Schalldämpfungs- | | |

## Patentansprüche

1. Medizinische Saugpumpe mit einem Pumpengehäuse (1, 8), mit einem im Pumpengehäuse (1, 8) angeordneten Pumpaggregat (6) zur Erzeugung eines Unterdrucks und mit einer Einrichtung zur Schalldämpfung, wobei die Einrichtung zur Schalldämpfung mindestens zwei elastische Lager (51, 52) zur elastischen Halterung des Pumpaggregats (6) relativ zum Pumpengehäuse (1, 8) aufweist, wobei die Lager (51, 52) beabstandet zueinander angeordnet sind, wobei das Pumpaggregat (6) in einem Schalldämpfungs-Gehäuse (4, 7) angeordnet ist und gegenüber diesem Schalldämpfungsgehäuse (4, 7) mittels der mindestens zwei Lager (51, 52) elastisch gehalten ist, wobei das Schalldämpfungsgehäuse (4, 7) lagefixiert im Pumpgehäuse (1, 8) angeordnet ist,
**dadurch gekennzeichnet,**
**dass** das Schalldämpfungsgehäuse (4, 7) bis auf eine erste und eine zweite Luftdurchlassöffnung (44, 45) und bis auf einen Vakuumanschluss (70) luftdicht geschlossen ausgebildet ist,
**dass** eine Luftaustauscheinheit (2) vorhanden ist, welche Luft durch die erste Luftdurchlassöffnung (44) des Schalldämpfungsgehäuses (4, 7) dem Pumpaggregat (6) zuführt und Luft durch die zweite Luftdurchlassöffnung (45) des Schalldämpfungsgehäuses (4, 7) aus dem Pumpaggregat (6) abführt, und
**dass** die Luftaustauscheinheit (2) zwischen Pumpgehäuse (1, 8) und Schalldämpfungsgehäuse (4, 7) angeordnet ist.

2. Saugpumpe nach Anspruch 1, wobei genau zwei oder genau drei elastische Lager (51, 52) vorhanden sind.

3. Saugpumpe nach einem der Ansprüche 1 oder 2, wobei die mindestens zwei Lager (51, 52) aus einem Elastomer gebildet sind.

4. Saugpumpe nach einem der Ansprüche 1 bis 3, wobei ein erstes und ein zweites Lager (51) dieser Lager in voneinander entgegengesetzte Richtungen ausgerichtet sind und ein drittes Lager (52) dieser Lager in eine weitere, von den genannten Richtungen senkrecht verlaufende Richtung ausgerichtet ist.

5. Saugpumpe nach Anspruch 4, wobei das erste und zweite Lager (51) im Bereich eines Vakuumaggregats (61) des Pumpaggregats (6) angeordnet sind und das dritte Lager (52) im Bereich eines Motors (60) des Pumpaggregats (6) angeordnet ist

6. Saugpumpe nach einem der Ansprüche 1 bis 5, wobei die mindestens zwei Lager (51, 52) in Lageraufnahmen (42, 43) einschiebbar gehalten sind.

7. Saugpumpe nach einem der Ansprüche 1 bis 6, wobei die mindestens zwei Lager (51, 52) aufsteckbare elastomere Körper sind.

8. Saugpumpe nach einem der Ansprüche 1 bis 7, wobei das Pumpaggregat (6) in einem Pumpaggregat-Träger (5) gehalten ist und wobei die mindestens zwei Lager (51, 52) an diesem Pumpaggregat-Träger (5) angeordnet sind.

9. Saugpumpe nach Anspruch 8, wobei das Pumpaggregat (6) unter Vorspannung im Pumpaggregat-Träger (5) gehalten ist.

10. Saugpumpe nach einem der Ansprüche 1 bis 9, wobei die Luftaustauscheinheit (2) plattenförmig ausgebildet ist und Kanäle (21, 22) mit Kanalwänden (23) aufweist, welche schalldämpfend ausgebildet sind.

11. Saugpumpe nach Anspruch 10, wobei die Kanalwände (23) weich ausgebildet sind.

12. Saugpumpe nach einem der Ansprüche 1 bis 11, wobei vom Pumpaggregat (6) weggeführte Abluft mindestens über einen Teil eines Strömungsweges durch einen rohrförmigen Luftstrom-Schalldämpfer (3) geleitet ist.

## Claims

1. Medical suction pump with a pump housing (1, 8), with a pump assembly (6) arranged in the pump housing (1, 8) and serving to generate an underpressure, and with a device for sound damping, wherein the device for sound damping has at least two elastic bearings (51, 52) for elastically supporting the pump assembly (6) relative to the pump housing (1, 8), wherein the bearings (51, 52) are arranged spaced apart from each other, wherein the pump assembly (6) is arranged in a sound-damping housing (4, 7) and is held elastically in relation to this sound-damping housing (4, 7) by means of the at least two bearings (51, 52), wherein the sound-damping housing (4, 7) is arranged in a fixed position in the pump housing (1, 8),
**characterized in**
**that** the sound-damping housing (4, 7) is closed in an airtight manner except for a first and a second air passage (44, 45) and except for a vacuum port (70),
**that** an air exchange unit (2) is present which delivers air to the pump assembly (6) through the first air passage (44) of the sound-damping housing (4, 7) and withdraws air from the pump assembly (6) through the second air passage (45) of the sound-damping housing (4, 7), and
**that** the air exchange unit (2) is arranged between pump housing (1, 8) and sound-damping housing (4, 7).

2. Suction pump according to Claim 1, wherein exactly two or exactly three elastic bearings (51, 52) are present.

3. Suction pump according to either of Claims 1 and 2, wherein the at least two bearings (51, 52) are formed from an elastomer.

4. Suction pump according to one of Claims 1 to 3, wherein a first and a second bearing (51) of these bearings are oriented in mutually opposite directions, and a third bearing (52) of these bearings is oriented in a further direction extending perpendicularly from said directions.

5. Suction pump according to Claim 4, wherein the first and second bearings (51) are arranged in the area of a vacuum assembly (61) of the pump assembly (6), and the third bearing (52) is arranged in the area of a motor (60) of the pump assembly (6)

6. Suction pump according to one of Claims 1 to 5, wherein the at least two bearings (51, 52) are held insertably in bearing seats (42, 43).

7. Suction pump according to one of Claims 1 to 6, wherein the at least two bearings (51, 52) are elastomer bodies that can be plugged on.

8. Suction pump according to one of Claims 1 to 7, wherein the pump assembly (6) is held in a pump assembly carrier (5), and wherein the at least two bearings (51, 52) are arranged on this pump assembly carrier (5).

9. Suction pump according to Claim 8, wherein the pump assembly (6) is held with pretensioning in the pump assembly carrier (5).

10. Suction pump according to one of Claims 1 to 9, wherein the air exchange unit (2) is plate-shaped and has channels (21, 22) with channel walls (23), which are designed to damp sound.

11. Suction pump according to claim10, wherein the channel walls (23) are pliable.

12. Suction pump according to one of Claims 1 to 11, wherein exhaust air withdrawn from the pump assembly (6) is conveyed at least over part of a flow path through a tubular airstream sound damper (3).

## Revendications

1. Pompe de succion médicale comprenant un boîtier de pompe (1, 8), une unité de pompe (6) disposée dans le boîtier de pompe (1, 8) pour générer une dépression et un dispositif d'atténuation du bruit, le dispositif d'atténuation du bruit présentant au moins deux paliers élastiques (51, 52) pour la fixation élastique de l'unité de pompe (6) par rapport au boîtier de pompe (1, 8), les paliers (51, 52) étant disposés à distance l'un de l'autre, l'unité de pompe (6) étant disposée dans un boîtier d'atténuation du bruit (4, 7) et étant fixée élastiquement par rapport à ce boîtier d'atténuation du bruit (4, 7) au moyen des au moins deux paliers (51, 52), le boîtier d'atténuation du bruit (4, 7) étant disposé dans le boîtier de pompe (1, 8) de manière fixée en position,
**caractérisée en ce que**
le boîtier d'atténuation du bruit (4, 7) est réalisé sous forme fermée de manière étanche à l'air à l'exception d'une première et d'une deuxième ouverture de passage d'air (44, 45) et à l'exception d'un raccordement au vide (70),
**en ce qu'**une unité d'échange d'air (2) est prévue, laquelle achemine de l'air à travers la première ouverture de passage d'air (44) du boîtier d'atténuation du bruit (4, 7) à l'unité de pompe (6) et évacue l'air à travers la deuxième ouverture de passage d'air (45) du boîtier d'atténuation du bruit (4, 7) hors de l'unité de pompe (6), et
**en ce que** l'unité d'échange d'air (2) est disposée entre le boîtier de pompe (1, 8) et le boîtier d'atténuation du bruit (4, 7).

2. Pompe de succion selon la revendication 1, dans laquelle exactement deux ou exactement trois paliers élastiques (51, 52) sont prévus.

3. Pompe de succion selon l'une quelconque des revendications 1 et 2, dans laquelle les au moins deux paliers (51, 52) sont formés à partir d'un élastomère.

4. Pompe de succion selon l'une quelconque des revendications 1 à 3, dans laquelle un premier et un deuxième palier (51) de ces paliers sont orientés dans des directions mutuellement opposées et un troisième palier (52) de ces paliers est orienté dans une direction supplémentaire s'étendant perpendiculairement aux dites directions.

5. Pompe de succion selon la revendication 4, dans laquelle le premier et le deuxième palier (51) sont disposés dans la région d'une unité de vide (61) de l'unité de pompe (6) et le troisième palier (52) est disposé dans la région d'un moteur (60) de l'unité de pompe (6).

6. Pompe de succion selon l'une quelconque des revendications 1 à 5, dans laquelle les au moins deux paliers (51, 52) sont retenus de manière insérable dans des logements de palier (42, 43).

7. Pompe de succion selon l'une quelconque des revendications 1 à 6, dans laquelle les au moins deux paliers (51, 52) sont des corps élastomères enfichables.

8. Pompe de succion selon l'une quelconque des revendications 1 à 7, dans laquelle l'unité de pompe (6) est retenue dans un support d'unité de pompe (5) et dans laquelle les au moins deux paliers (51, 52) sont disposés au niveau de ce support d'unité de pompe (5).

9. Pompe de succion selon la revendication 8, dans laquelle l'unité de pompe (6) est retenue avec précontrainte dans le support d'unité de pompe (5).

10. Pompe de succion selon l'une quelconque des revendications 1 à 9, dans laquelle l'unité d'échange d'air (2) est réalisée sous forme de plaque et présente des canaux (21, 22) avec des parois de canaux (23) qui sont réalisées de manière à atténuer le bruit.

11. Pompe de succion selon la revendication 10, dans laquelle les parois de canaux (23) sont réalisées sous forme souple.

12. Pompe de succion selon l'une quelconque des revendications 1 à 11, dans laquelle l'air d'évacuation guidé hors de l'unité de pompe (6) est conduit sur au moins une partie d'une voie d'écoulement à travers un atténuateur de bruit de flux d'air de forme tubulaire (3).
